(19) Europäisches Patentamt
European Patent Office
Office européen des brevets

(11) Numéro de publication: **0 034 838**
**A2**

(12) **DEMANDE DE BREVET EUROPEEN**

(21) Numéro de dépôt: **81103269.7**

(22) Date de dépôt: **13.07.79**

(51) Int. Cl.³: **C 07 C 69/65**
C 07 C 67/14

(30) Priorité: **24.07.78 FR 7821849**
**28.05.79 FR 7913532**

(43) Date de publication de la demande:
**02.09.81 Bulletin 81/35**

(84) Etats contractants désignés:
**AT DE IT NL SE**

(60) Numéro de publication de la demande initiale en application de l'article 76 CBE: **0 008 256**

(71) Demandeur: **HEXACHIMIE Société anonyme dite:**
**128, rue Danton**
**F-92500 Rueil-Malmaison(FR)**

(72) Inventeur: **Teulon, Jean-Marie Charles**
**12, Résidence du Bel Ebat 56, avenue de Verdun**
**F-78170 La Celle Saint Cloud(FR)**

(74) Mandataire: **de Haas, Michel**
**Cabinet Beau de Lomenie 55 rue d'Amsterdam**
**F-75000 Paris 8ème(FR)**

(54) **Nouveaux esters halogénoéthyliques d'acide indane acétique, et leur préparation.**

(57) L'invention concerne de nouveaux composés de formule:

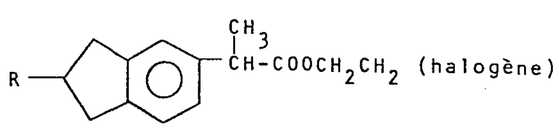

(R = éthyle ou isopropyle)

Préparation par réaction du chlorure d'acide correspondant sur l'halogénoalcool adéquat.

Intermédiaires de la synthèse d'esters aminés analgésiques correspondants.

EP 0 034 838 A2

<u>Nouveaux esters halogénoéthyliques d'acide indane acétique, et leur préparation.</u>

La présente invention concerne de nouveaux composés de formule

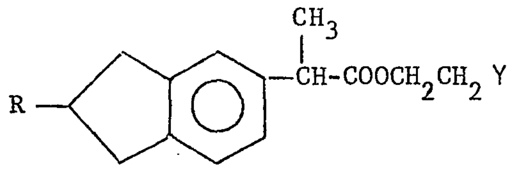

(V)

(R = éthyle ou isopropyle) (Y = atome d'halogène)

Leur préparation est effectuée, selon le schéma suivant :

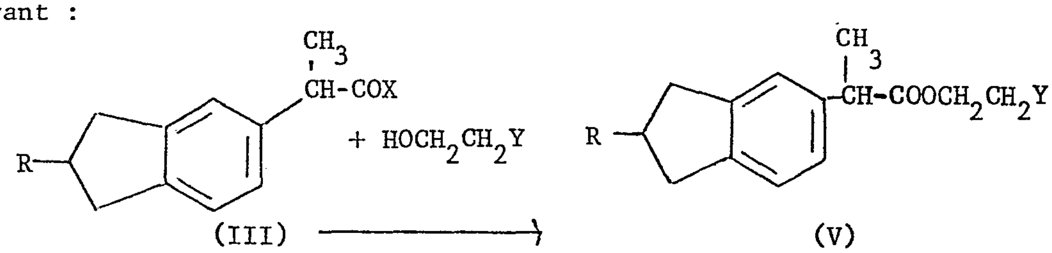


(III) (V)

(X = atome d'halogène)
par réaction de l'halogénure d'acide (III) correspondant, de préférence le chlorure, sur l'halogénoalcool adéquat.

On fait réagir les halogénures d'acide de formule III avec un halogéno-alcool $HOCH_2CH_2Y$ dans un solvant organique comme l'acétone, le chloroforme, le chlorure de méthylène ou un hydrocarbure aromatique en présence ou non d'une base comme la pyridine ou la triéthylamine.

Ces nouveaux composés (V) sont utiles dans la synthèse d'esters aminés (I) analgésiques, selon le schéma suivant :

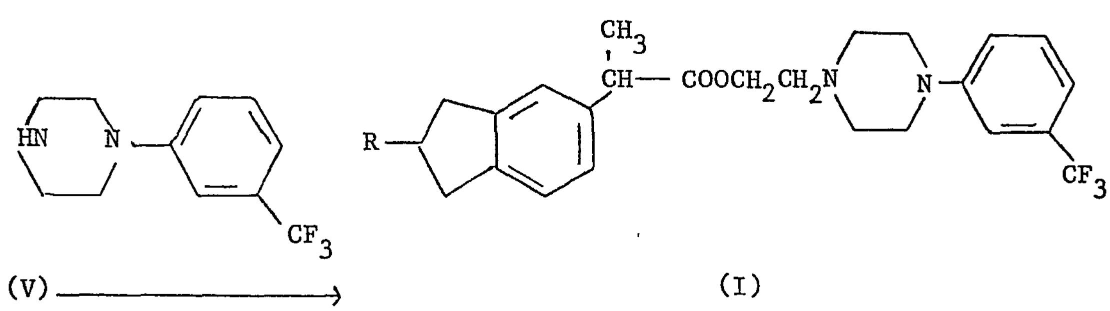

(V) (I)

Les exemples suivants illustrent l'invention sans toutefois en limiter la portée.

Exemple 1

Chlorure de l'acide méthyl-2[(éthyl-2)indanyl-5]acétique.

Une solution de 57 g d'acide méthyl-2[(éthyl-2)indanyl-5]acétique, 40 ml de chlorure de thionyle dans 200 ml de benzène est portée au reflux durant 2 heures. Le solvant et l'excès de chlorure de thionyle sont ensuite évaporés sous vide. L'huile obtenue est ensuite soumise à distillation sous vide. On récupère ainsi 56 g de chlorure de l'acide méthyl-2[(éthyl-2)indanyl-5]acétique sous forme d'un liquide.

$Eb_{(1mmHg)}$ = 125-128°C.

Exemple 2

Chlorure de l'acide méthyl-2[(isopropyl-2)indanyl-5]acétique.

Selon le mode opératoire de l'exemple 1 mais en utilisant 31 g d'acide méthyl-2[(isopropyl-2)indanyl-5]acétique on récupère 30 g de chlorure de l'acide méthyl-2[(isopropyl-2) indanyl-5] acétique sous forme d'un liquide :

$Eb_{(1 mmHg)}$ = 150°C.

Exemple 3

Ester bromo éthylique de l'acide méthyl-2[(éthyl-2)indanyl-5]acétique.

Formule V R = éthyle Y = Br

Méthode A :

On porte au reflux durant 4 heures la solution de 4,4 g de chlorure de l'acide méthyl-2[(éthyl-2)indanyl-5]acétique et 2,45 g de bromo-2 éthanol dans 50 ml d'acétone anhydre.

Après évaporation du solvant, le résidu huileux obtenu est dissous dans l'éther qu'on lave en présence de glace, avec une solution aqueuse de bicarbonate de sodium, à l'eau puis qu'on sèche sur sulfate de sodium. Après filtration et évaporation du solvant on obtient 5,9 g d'ester bromo éthylique de l'acide méthyl-2[(éthyl-2)indanyl-5]acétique sous forme d'un résidu huileux qu'on utilise brut pour l'étape suivante.

Méthode B

On additionne à 0°C une solution de 12 g de chlorure de l'acide méthyl-2[(éthyl-2)indanyl-5]acétique dans 15 ml de chloroforme à une solution de 6,4 g de bromo-2 éthanol et 5,5 ml de pyridine dans 30 ml de chloroforme.

Après la fin de l'addition, on porte le mélange réactionnel au reflux durant 1 heure.

Après refroidissement, le mélange réactionnel est lavé à l'eau, avec de l'acide chlorhydrique 5% puis encore à l'eau. La phase chloroformique est séchée et après évaporation du solvant le résidu huileux est soumis à distillation sous vide. On récupère ainsi 13,7 g de l'ester bromo éthylique de l'acide méthyl-2[(éthyl-2)indanyl-5]acétique sous forme d'une huile.

$Eb_{0,2 \text{ mm de Hg}} = 138\text{-}148°C$.

Exemple 4

Ester bromoéthylique de l'acide méthyl-2[isopropyl-2)indanyl-5] acétique.

formule V R = isopropyle Y = Br

Méthode A :

On additionne à 0°C une solution de 32 g de chlorure de l'acide méthyl-2[(isopropyl-2)indanyl-5]acétique dans 25 ml de chloroforme à une solution de 16 g de bromo-2 éthanol et 14 ml de pyridine dans 50 ml de chloroforme.

Après la fin de l'addition, on porte le mélange réactionnel au reflux durant 1 heure.

Après refroidissement le mélange réactionnel est lavé à l'eau, avec de l'acide chlorhydrique 5 % puis encore à l'eau.

La phase chloroformique est séchée et après évaporation du solvant le résidu huileux 49 g est soumis à distillation sous vide. On récupère ainsi 35,2 g de l'ester bromoéthylique de l'acide méthyl-2[(isopropyl-2)indanyl-5]acétique sous forme d'une huile.

$Eb_{1,5 \text{ mm Hg}} = 165\text{-}175°C$.

Méthode B :

On additionne goutte à goutte une solution de 32 g de chlorure de l'acide méthyl-2[(isopropyl-2)indanyl-5]acétique dans 25 ml d'acétone à une solution de 16 g de bromo-2 éthanol et 10,4 ml de pyridine dans 125 ml d'acétone.

Après la fin de l'addition, on porte le mélange réactionnel au reflux durant 1 h 30.

Le mélange réactionnel est ensuite concentré sous vide, repris à l'éther qu'on lave à l'eau, avec de l'acide chlorhydrique 5 % puis encore à l'eau. La phase éthérée séchée, le solvant est évaporé sous vide.

On récupère ainsi 40 g de l'ester bromoéthylique de l'acide méthyl-2[(isopropyl-2)indanyl-5]acétique sous forme d'une huile.

## R E V E N D I C A T I O N S

1. Nouveaux composés caractérisés en ce qu'il répondent à la formule :

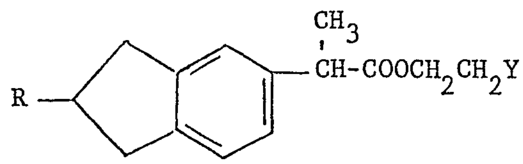

(V)

dans laquelle R représente un groupe éthyle ou isopropyle et Y représente un atome d'halogène.

2. Nouveaux composés selon la revendication 1, caractérisés en ce que Y représente le brome.

3. Procédé de préparation des composés selon la revendication 1 ou 2, caractérisé en ce que l'on fait réagir un composé de formule

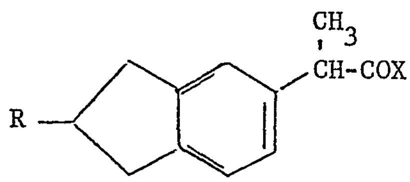

(III)

(X = halogène)

sur un halogénoalcool $OHCH_2CH_2Y$ dans un solvant organique.

4. Procédé selon la revendication 3, caractérisé en ce que le solvant est choisi parmi l'acétone, le chloroforme, le chlorure de méthylène ou un hydrocarbure aromatique.

5. Procédé selon la revendication 3 ou 4, caractérisé en ce que l'on effectue la réaction en présence d'une base comme la pyridine ou la triéthylamine.